(19) Europäisches Patentamt
European Patent Office
Office européen des brevets

(11) Veröffentlichungsnummer: **0 006 169**
**A1**

(12) # EUROPÄISCHE PATENTANMELDUNG

(21) Anmeldenummer: **79101727.0**

(22) Anmeldetag: **01.06.79**

(51) Int. Cl.³: **C 07 D 263/04**, A 01 N 43/76, C 07 D 263/06

(30) Priorität: **05.06.78 DE 2824540**

(43) Veröffentlichungstag der Anmeldung: **09.01.80 Patentblatt 80/1**

(84) Benannte Vertragsstaaten: **BE CH DE FR GB IT NL**

(71) Anmelder: **Henkel Kommanditgesellschaft auf Aktien, Postfach 1100, D-4000 Düsseldorf 1 (DE)**

(72) Erfinder: **Bansemir, Klaus, Dr., Ursulaweg 51, D-4018 Langenfeld (DE)**
Erfinder: **Bellinger, Horst, Dr., Schumannstrasse 83, D-4000 Düsseldorf (DE)**
Erfinder: **Disch, Karlheinz, Dr., Holbeinstrasse 10, D-5657 Haan/Rhld (DE)**
Erfinder: **Stracke, Heinz-Ulrich, Dr., Blumenstrasse 36, D-4018 Langenfeld (DE)**
Erfinder: **Syldatk, Andreas, Dr., Kolhagenstrasse 44, D-4000 Düsseldorf 13 (DE)**

(54) **3-(omega-Aminoalkyl)-substituierte 1,3-Oxazolidine und ihre Verwendung als antimikrobielle Mittel.**

(57) Antimikrobiell wirksame substituierte Oxazolidine der Formel

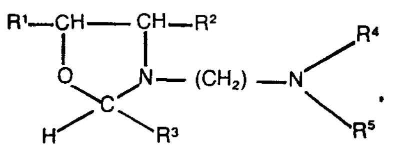

(I)

in der $R^1$ und $R^2$ Alkylreste mit 1 bis 17 Kohlenstoffatomen bedeuten, wobei die Summe der Kohlenstoffatome in $R^1$ und $R^2$ 8 bis 18 beträgt, $R^3$ Wasserstoff, einen Alkylrest mit 1 bis 5 Kohlenstoffatomen, einen gegebenenfalls durch Halogen, eine Hydroxyl- oder Methoxygruppe substituierten Phenylrest oder die Gruppe $C_6H_5CH{=}CH-$ darstellt, n eine ganze Zahl von 2 bis 6 und $R^4$ und $R^5$ unabhängig voneinander Wasserstoff oder einen Alkylrest mit 1 bis 3 Kohlenstoffatomen bedeuten.

EP 0 006 169 A1

4000 Düsseldorf, den 28.5.1979
Henkelstraße 67

HENKEL KGaA 0006169
ZR-FE/Patente

Dr. Gla/B

P a t e n t a n m e l d u n g

D 5655

BEZEICHNUNG GEÄNDERT
siehe Titelseite

"3-(omega-Aminoalkyl)-substituierte 1,3-Oxazolidine, deren Herstellung und Verwendung als antimikrobielle Mittel"

Die Erfindung betrifft 3-(omega-Aminoalkyl)-1,3-oxazolidine, die in 3- und 4-Stellung durch Alkylreste substituiert sind, ihre Herstellung und ihre Verwendung als antimikrobielle Mittel.

Gegenstand der Erfindung sind substituierte Oxazolidine der Formel

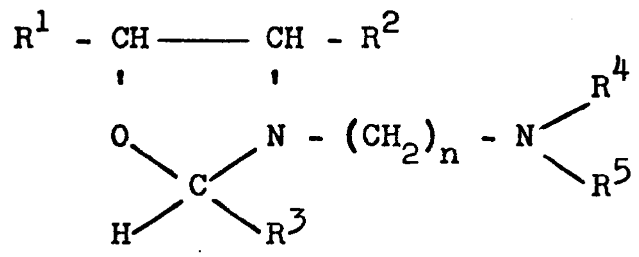

(I)

in der $R^1$ und $R^2$ Alkylreste mit 1 bis 17 Kohlenstoffatomen bedeuten, wobei die Summe der Kohlenstoffatome in $R^1$ und $R^2$ 8 bis 18 beträgt, $R^3$ Wasserstoff, einen Alkylrest mit 1 bis 5 Kohlenstoffatomen, einen gegebenenfalls durch Halogen, eine Hydroxyl- oder Methoxygruppe substituierten Phenylrest oder die Gruppe $C_6H_5CH=CH-$ darstellt, n eine ganze Zahl von 2 bis 6 und $R^4$ und $R^5$ unabhängig voneinander Wasserstoff oder einen Alkylrest mit 1 bis 3 Kohlenstoffatomen bedeuten. Es wurde gefunden, daß diese substituierten Oxazolidine sich mit ausgezeichneter Wirkung als antimikrobielle Wirkstoffe verwenden lassen.

/2

Gegenstand der Erfindung ist ferner ein Verfahren zur Herstellung der oben definierten Oxazolidine, bei dem man Hydroxyalkylamine der Formel

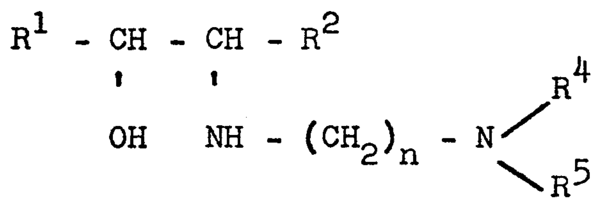

(II)

in der $R^1$, $R^2$, $R^4$, $R^5$ und n die für die Formel I angegebene Bedeutung haben, und Aldehyde der Formel

$$R^3 - CHO \qquad (III)$$

in der $R^3$ die für die Formel I angegebene Bedeutung hat, im Molverhältnis 1 : 1 unter Wasserabspaltung miteinander umsetzt. Dieses Verfahren wird in Analogie zu der von M. Senkus in Journal of the American Chemical Society, Band 67 (1945), Seiten 1515 - 1519 beschriebenen Darstellung von Oxazolidinen anderer Struktur durchgeführt. Die Reaktionspartner werden dabei zweckmäßigerweise in Lösungsmitteln, die mit Wasser azeotrope Gemische bilden,an einem Wasserabscheider solange zum Rückflußkochen erhitzt, bis die zu erwartende Menge Wasser übergegangen ist.

Die als Ausgangsmaterial verwendeten Hydroxyalkylamine der Formel II sind nach einem in der DE-OS 25 20 267 offenbarten Verfahren zugänglich. Entsprechend der dort beschriebenen Arbeitsweise geht man aus von Gemischen von Olefinen mit 10 bis 20 Kohlenstoffatomen und statistisch verteilten innenständigen Doppelbindungen. Solche Olefingemische können beispielsweise durch katalytische Dehydrierung oder durch Chlorierung/Dehydrochlorierung von $C_{10}$-$C_{20}$-Paraffinen und selektive Extraktion der erhaltenen innenständigen Monoolefine hergestellt werden.

/3

Als Gemische von isomeren Monoolefinen sind die Fraktionen mit hohem Gehalt an linearen $C_{11}$-$C_{14}$- und $C_{15}$-$C_{18}$-Olefinen bevorzugt. Die besonders bevorzugten Gemische von innenständigen Olefinen haben folgende Kettenlängenverteilung:

a) Fraktion $C_{11}$-$C_{14}$ (Verteilung in Gewichtsprozent)

| | |
|---|---|
| $C_{11}$-Olefine | ca. 22 |
| $C_{12}$- " | ca. 30 |
| $C_{13}$- " | ca. 26 |
| $C_{14}$- " | ca. 22 |

b) Fraktion $C_{15}$-$C_{18}$

| | |
|---|---|
| $C_{15}$-Olefine | ca. 26 |
| $C_{16}$- " | ca. 35 |
| $C_{17}$- " | ca. 31 |
| $C_{18}$- " | 6 |

Die anteilmäßige Kettenlängenverteilung kann aber auch hiervon abweichen.

Zur Herstellung der Hydroxyalkylamine der Formel II werden die Olefingemische nach bekannten Verfahren, z.B. mit Peressigsäure epoxidiert. Anschließend werden die erhaltenen Epoxidgemische mit den entsprechenden Diaminen wie Äthylendiamin, Trimethylendiamin, Tetramethylendiamin, Pentamethylendiamin, Hexamethylendiamin oder deren N-Alkyl- bzw. N,N-Dialkylderivaten umgesetzt. Dabei werden die Diamine in 1- bis 15-facher molarer Menge, bezogen auf das Epoxidgemisch, eingesetzt. Dem Amin-Epoxid-Gemisch kann gegebenenfalls ein katalytisch wirksames Lösungsmittel wie Wasser, Äthanol oder Glycerin zugesetzt werden. Die Reaktion wird bei 100 bis 230°C oder 373 bis 503 K, gegebenenfalls im Autoklaven, durchgeführt. Nach beendeter Reaktion wird das erhaltene Gemisch nach be-

/4

kannten Verfahren, normalerweise durch Destillation aufgearbeitet.

Bei den weiterhin als Ausgangsmaterial verwendeten Aldehyden der Formel III handelt es sich um bekannte Substanzen. Entsprechend der Bedeutung von $R^3$ kommt Formaldehyd, Acetaldehyd, Propionaldehyd, Butyraldehyd, Valeraldehyd, Benzaldehyd, die isomeren Chlor- und Dichlorbenzaldehyde, die isomeren Hydroxybenzaldehyde, die isomeren Methoxybenzaldehyde und Zimtaldehyd in Frage.

Die erfindungsgemäßen Substanzen stellen in der Regel keine einheitlichen Verbindungen dar; gemäß ihrer Herstellung handelt es sich normalerweise um Gemische von Oxazolidinen, die sich durch die verschiedene Kettenlänge der Alkylsubstituenten in der 4- und 5-Stellung unterscheiden. Aus diesem Grunde lassen sich die erfindungsgemäßen Oxazolidine am besten über die bei der Herstellung verwendeten Epoxyalkane, Diamine und Aldehyde charakterisieren. Als Beispiel für erfindungsgemäße Substanzen werden die Umsetzungsprodukte von innenständigem $C_{11}$-$C_{14}$-Epoxyalkan, Äthylendiamin und Formaldehyd, innenständigem $C_{11}$-$C_{14}$-Epoxyalkan, Trimethylendiamin und Acetaldehyd, innenständigem $C_{11}$-$C_{14}$-Epoxyalkan, Pentamethylendiamin und Butyraldehyd, innenständigem $C_{11}$-$C_{14}$-Epoxyalkan, Hexamethylendiamin und Benzaldehyd, innenständigem $C_{11}$-$C_{14}$-Epoxyalkan, N,N-Dimethyläthylendiamin und p-Chlorbenzaldehyd, innenständigem $C_{14}$-$C_{16}$-Epoxyalkan, N,N-Diäthyltetramethylendiamin und Formaldehyd, innenständigem $C_{14}$-$C_{16}$-Epoxid, Pentamethylendiamin und Zimtaldehyd, innenständigem $C_{15}$-$C_{18}$-Epoxyalkan, Äthylendiamin und Propionaldehyd, innenständigem $C_{15}$-$C_{18}$-Epoxyalkan, Trimethylendiamin und p-Hydroxybenzaldehyd, innenständigem $C_{15}$-$C_{18}$-Epoxyalkan, Tetramethylendiamin und o-Hydroxybenzaldehyd genannt.

/5

Von besonderem Interesse sind die durch Umsetzung von $C_{11}$-$C_{14}$-Epoxyalkanen und $C_{15}$-$C_{18}$-Epoxyalkanen mit Äthylendiamin, Propylendiamin, N,N-Dimethylendiamin, Hexamethylendiamin und nachfolgende Reaktion mit Formaldehyd, Acetaldehyd, Propionaldehyd, Benzaldehyd, p-Methoxybenzaldehyd und Zimtaldehyd erhaltenen Oxazolidine.

Zur Verwendung in antimikrobiellen Mitteln können die erfindungsgemäßen Oxazolidine in flüssige, pastenförmige oder feste Zubereitungen eingearbeitet werden, z.B. in wässrige Lösungen, Suspensionen, Emulsionen und Lösungen in organischen Lösungsmitteln. Derartige antimikrobielle Mittel können auf den verschiedensten Gebieten zum Einsatz gelangen, z.B. als Reinigungs-, Desinfektions- und Konservierungsmittel für Textilien, Fußböden, Krankenhauseinrichtungen und -instrumente, gewerbliche Betriebe, wie Molkereien, Brauereien und Wäschereien. In antimikrobiellen Mitteln werden die erfindungsgemäßen Oxazolidine in Mengen von 0,1 bis 25 Gew.-%, vorzugsweise 0,5 bis 20Gew.-%, bezogen auf das gesamte antimikrobielle Mittel, eingesetzt.

Außerdem können die erfindungsgemäßen Oxazolidine zur Konservierung von technischen Produkten, die dem Befall durch Bakterien und Pilze oder sonstiger mikrobieller Zerstörung unterliegen, wie beispielsweise Stärkekleistern, Leimen, Dispersionsfarben sowie Schneid- und Bohrölen, dienen. Für diesen Verwendungszweck ist im allgemeinen ein Zusatz von 0,1 bis 2 Gew.-%, bezogen auf das zu konservierende Material, ausreichend.

Die nachfolgenden Beispiele sollen den Gegenstand der Erfindung näher erläutern, ohne ihn hierauf zu beschränken.

Beispiele

In die Untersuchungen auf bakteriostatische und bakterizide Wirksamkeit wurden die in der nachstehenden Tabelle I aufgeführten Substanzen der Formel

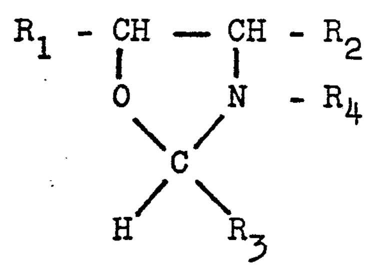

einbezogen. Die Herstellung der Substanzen erfolgte nach den Angaben in Journal of the American Chemical Society, Band 67 (1945), Seiten 1515 - 1519. Unter Verwendung von Toluol als Lösungsmittel wurden Mischungen aus einem entsprechenden Aminoalkohol und einem entsprechenden Aldehyd im Molverhältnis 1 : 1 am Wasserabscheider so lange zum Rückflußkochen erhitzt, bis sich die berechnete Menge Wasser abgeschieden hatte. Die nach dem Abdestillieren des Lösungsmittels zurückbleibenden Produkte wurden ohne weitere Reinigung verwendet.

/7

Tabelle I

| Substanz | $R_1 + R_2$ | $R_3$ | $R_4$ | Brechungsindex $n_D^{20}$ |
|---|---|---|---|---|
| A 1 | $C_9-C_{12}$ | H | $-CH_2CH_2NH_2$ | 1,4750 |
| A 2 | " | $CH_3$ | " | 1,4693 |
| A 3 | " | $C_3H_7$ | " | 1,4690 |
| A 4 | " | $C_6H_5$ | " | 1,5030 |
| A 5 | " | $p-CH_3OC_6H_5$ | " | 1,5100 |
| A 6 | " | $C_6H_5CH=CH$ | " | 1,5113 |
| B 1 | " | H | $-CH_2CH_2CH_2NH_2$ | 1,4845 |
| B 2 | " | $CH_3$ | " | 1,4818 |
| B 3 | " | $C_3H_7$ | " | 1,4758 |
| B 4 | " | $C_6H_5$ | " | 1,5103 |
| B 5 | " | $p-CH_3C_6H_5$ | " | 1,5161 |
| B 6 | " | $C_6H_5CH=CH$ | " | 1,5261 |
| C 1 | " | H | $-(CH_2)_6NH_2$ | 1,4797 |
| C 2 | " | $CH_3$ | " | 1,4780 |
| C 3 | " | $C_3H_7$ | " | 1,4720 |
| C 4 | " | $C_6H_5$ | " | 1,5074 |
| C 5 | " | $p-CH_3OC_2H_5$ | " | 1,5143 |
| C 6 | " | $C_6H_5CH=CH$ | " | 1,5216 |

/8

Tabelle I - Fortsetzung

| Substanz | $R_1 + R_2$ | $R_3$ | $R_4$ | Brechungsindex $n_D^{20}$ |
|---|---|---|---|---|
| D 1 | $C_{13}$-$C_{16}$ | H | $-CH_2CH_2NH_2$ | 1,4840 |
| D 2 | " | $CH_3$ | " | 1,4800 |
| D 3 | " | $C_3H_7$ | " | 1,4779 |
| D 4 | " | $C_6H_5$ | " | 1,5087 |
| D 5 | | p-$CH_3OC_6H_5$ | $-CH_2CH_2NH_2$ | 1,5124 |
| D 6 | " | $C_6H_5CH=CH$ | " | 1,5178 |
| E 1 | " | H | $-CH_2CH_2CH_2NH_2$ | 1,4802 |
| E 2 | " | $CH_3$ | " | 1,4745 |
| E 3 | " | $C_3H_7$ | " | 1,4738 |
| E 4 | " | $C_6H_5$ | " | 1,5054 |
| E 5 | " | p-$CH_3OC_6H_5$ | " | 1,5119 |
| E 6 | " | $C_6H_5CH=CH$ | " | 1,5180 |
| F 1 | " | H | $-CH_2-CH_2-CH_2N(CH_3)_2$ | 1,4650 |
| F 2 | " | $CH_3$ | " | 1,4619 |
| F 3 | " | $C_3H_7$ | " | 1,4635 |
| F 4 | " | $C_6H_5$ | " | 1,4880 |
| F 5 | " | p-$CH_3OC_6H_5$ | " | 1,4990 |
| F 6 | " | $C_6H_5CH=CH$ | " | 1,5038 |
| G 1 | " | H | $-(CH_2)_6NH_2$ | 1,4790 |
| G 2 | " | $CH_3$ | " | 1,4750 |
| G 3 | " | $C_3H_7$ | " | 1,4722 |
| G 4 | " | $C_6H_5$ | " | 1,5017 |
| G 5 | " | p-$CH_3OC_6H_5$ | " | 1,5093 |
| G 6 | " | $C_6H_5CH=CH$ | " | 1,5153 |

/9

Beispiel 1

Die mikrobistatische Wirksamkeit der Substanzen A 1 bis G 6 wurde gegenüber folgenden Testkeimsuspensionen bestimmt:

| | |
|---|---|
| 1) Staphylococcus aureus | $5 \times 10^7$ Keime/ml |
| 2) Escherichia coli | $5 \times 10^7$ Keime/ml |
| 3) Pseudomonas aeruginosa | $5 \times 10^7$ Keime/ml |
| 4) Candida albicans | $5 \times 10^7$ Keime/ml |

Die Hemmkonzentrationen der zu untersuchenden Produkte wurde mit Hilfe des Verdünnungstestes nach den Richtlinien für die Prüfung chemischer Desinfektionsmittel der Deutschen Gesellschaft für Hygiene und Mikrobiologie (1959) ermittelt.

Die zu prüfenden Substanzen wurden in wenig Äthanol gelöst. Dabei wurde nur so viel Äthanol verwendet, daß die Alkoholkonzentration in den später hergestellten Prüflösungen höchstens 1 Gew.-% betrug. Durch Verdünnen mit entionisiertem Wasser wurden Standardlösungen mit bekanntem Wirkstoffgehalt hergestellt. Die für die vorgesehenen Prüfkonzentrationen erforderlichen Mengen Standardlösung wurden in Reagenzröhrchen pipettiert, die mit jeweils 2 ml Standard-I-Bouillon oder Bierwürze ($8^o$ BG) beschickt waren. Anschließend wurde jeweils so viel entionisiertes Wasser zugegeben, daß die Röhrchen 10 ml Flüssigkeit enthielten. Auf diese Weise wurden Nährlösungen mit folgenden Wirkstoffkonzentrationen (in pp,) hergestellt:

1000, 750, 500, 250, 100, 50, 25, 10, 5, 2,5, 1.

In diese Nährlösungen wurde jeweils 0,1 ml Testkeimsuspension der angegebenen Konzentration gebracht. Die mit Bakterien geimpften Proben wurden 3 Tage lang bei $37^o$ C

/10

oder 310 K im Brutschrank aufbewahrt. Die mit Candida albicans geimpften Proben wurden 6 Tage lang bei 30° C oder 303 K bebrütet. Danach wurde festgestellt, welche dem Nährmedium zugefügte Wirkstoffkonzentration das Wachstum der Keime gerade noch gehemmt hatte. Der auf diese Weise gefundene Wert wurde als Hemmkonzentration bezeichnet. Die für die Produkte A 1 bis G 6 gefundenen Hemmkonzentrationen sind in der Tabelle II wiedergegeben.

Tabelle II

Hemmkonzentration der Produkte A 1 bis G 6 in ppm

| Produkt | Hemmkonzentration in ppm | | | |
|---|---|---|---|---|
| | Testkeim | | | |
| | 1 | 2 | 3 | 4 |
| A 1 | 25 | 100 | 1000 | 500 |
| A 2 | 50 | 100 | 250 | 250 |
| A 3 | 50 | 100 | 250 | 250 |
| A 4 | 50 | 100 | 500 | 250 |
| A 5 | 50 | 250 | 500 | 250 |
| A 6 | 50 | 250 | 1000 | 100 |
| B 1 | 5 | 50 | 50 | 50 |
| B 2 | 5 | 50 | 50 | 50 |
| B 3 | 50 | 50 | 100 | 500 |
| B 4 | 50 | 50 | 100 | 250 |
| B 5 | 50 | 50 | 100 | 100 |
| B 6 | 5 | 50 | 100 | 100 |
| C 1 | 50 | 250 | 750 | 250 |
| C 2 | 50 | 100 | 750 | 250 |
| C 3 | 50 | 250 | > 1000 | 250 |
| C 4 | 50 | 100 | > 1000 | 250 |
| C 5 | 50 | 250 | 1000 | 250 |
| C 6 | 10 | 100 | 100 | 100 |
| D 1 | 2,5 | 5 | 100 | 50 |
| D 2 | 2,5 | 50 | 50 | 50 |

Tabelle II - Fortsetzung

| Produkt | Hemmkonzentration in ppm | | | |
|---|---|---|---|---|
| | Testkeim | | | |
| | 1 | 2 | 3 | 4 |
| D 3 | 50 | 50 | 100 | 50 |
| D 4 | 50 | 50 | 100 | 50 |
| D 5 | 5 | 5 | 100 | 50 |
| D 6 | 5 | 50 | 1000 | 50 |
| E 1 | 1 | 2,5 | 50 | 50 |
| E 2 | 1 | 2,5 | 50 | 50 |
| E 3 | 1 | 2,5 | 5 | 50 |
| E 4 | 50 | 50 | 50 | 50 |
| E 5 | 1 | 50 | 50 | 50 |
| E 6 | < 1 | 2,5 | 2,5 | 50 |
| F 1 | 1 | 1 | 500 | 50 |
| F 2 | 50 | 50 | 500 | 100 |
| F 3 | 2,5 | 50 | 1000 | 50 |
| F 4 | 50 | 50 | > 1000 | 50 |
| F 5 | 50 | 50 | 750 | 50 |
| F 6 | 50 | 50 | > 1000 | 100 |
| G 1 | 5 | 7,5 | 500 | 50 |
| G 2 | 2,5 | 5 | 500 | 10 |
| G 3 | 2,5 | 5 | 500 | 10 |
| G 4 | 2,5 | 7,5 | 500 | 50 |
| G 4 | 2,5 | 5 | 500 | 100 |
| G 6 | 2,5 | 10 | 500 | 100 |

/12

Beispiel 2

Die mikrobizide Wirkung der Substanzen A 1 bis G 6 wurde gegenüber folgenden Testkeimsuspensionen bestimmt:

1) Staphylococcus aureus $2 \times 10^8$ Keime/ml
2) Escherichia coli $3 \times 10^8$ Keime/ml
3) Pseudomonas aeruginosa $2 \times 10^8$ Keime/ml
4) Candida albicans $8 \times 10^7$ Keime/ml

Die Abtötungszeiten der zu untersuchenden Produkte wurden mit Hilfe des Suspensionstests nach den Richtlinien für die Prüfung chemischer Desinfektionsmittel der Deutschen Gesellschaft für Hygiene und Mikrobiologie (1959) ermittelt.

Die zu prüfenden Substanzen wurden zunächst in wenig Aceton gelöst. Aus den acetonischen Lösungen wurden durch Verdünnen mit entionisiertem Wasser Testlösungen hergestellt, die 1000 ppm, 750 ppm, 500 ppm, 250 ppm, 100 ppm und 50 ppm Wirkstoff und maximal 1 Gew.-% Aceton enthielten.

Den Richtlinien entsprechend wurden bei Raumtemperatur jeweils 0,1 ml Testkeimsuspension in Reagenzgläser pipettiert. Hierzu wurden jeweils 10 ml der oben beschriebenen Testlösungen gegeben. Nach Einwirkungszeiten von 2 1/2, 5, 10, 20, 40, 60 und 120 Minuten wurde den Reagenzgläsern mit Hilfe einer Öse ein Tropfen Material entnommen und in 10 ml Nährlösung, die 3 % Tween 80 und 0,3 % Lecithin als Enthemmer enthielt, überimpft. Das Nährmedium bestand bei den Bakterien aus 1 gew.-%iger Standard-I-Bouillon (Merck), bei Candida albicans aus 1 gew.-%iger Bierwürzelösung. Die mit Bakterien beimpften Proben wurden bei 37° C oder 310 K, die mit Candida albicans beimpften Proben bei 30° C oder 303 K bebrütet. Nach frühestens 3 Tagen wurden die Kulturen makroskopisch

/13

auf Wachstum beurteilt und auf diesem Weg die Abtötungszeiten ermittelt, die in der nachstehenden Tabelle III wiedergegeben sind.

Tabelle III

Abtötungszeiten der Produkte A 1 bis G 6 bei Konzentrationen von 500 ppm, 250 ppm, 100 ppm und 50 ppm in Minuten

| Substanz | ppm | Abtötungszeit in Minuten | | | |
|---|---|---|---|---|---|
| | | 1 | 2 | 3 | 4 |
| A 1 | 1000 | - | - | 5 | 2,5 |
| | 750 | - | - | > 120 | 5 |
| | 500 | - | 20 | - | 20 |
| | 250 | 40 | 60 | - | - |
| | 100 | > 120 | > 120 | - | - |
| | 50 | > 120 | - | - | - |
| A 2 | 500 | - | 20 | 20 | 10 |
| | 250 | 5 | 20 | 40 | 60 |
| | 100 | 40 | 60 | > 120 | > 120 |
| | 50 | > 120 | - | - | - |
| A 3 | 1000 | - | - | 2,5 | - |
| | 750 | - | - | 10 | - |
| | 500 | - | - | 20 | 10 |
| | 250 | 10 | 10 | - | 40 |
| | 100 | 60 | > 120 | - | > 120 |
| | 50 | > 120 | > 120 | - | - |
| A 4 | 1000 | - | - | 5 | 2,5 |
| | 750 | - | - | 5 | 2,5 |
| | 500 | - | 10 | 20 | 60 |
| | 250 | 40 | 60 | - | - |
| | 100 | > 120 | > 120 | - | - |
| | 50 | > 120 | - | - | - |

Tabelle III - Fortsetzung

| Substanz | ppm | Abtötungszeit in Minuten | | | |
|---|---|---|---|---|---|
| | | 1 | 2 | 3 | 4 |
| A 5 | 1000 | - | - | 10 | - |
| | 750 | - | - | 20 | 120 |
| | 500 | - | 20 | 120 | > 120 |
| | 250 | 120 | > 120 | - | > 120 |
| | 100 | > 120 | > 120 | - | - |
| | 50 | > 120 | - | - | - |
| A 6 | 1000 | - | - | 120 | - |
| | 750 | - | - | > 120 | - |
| | 500 | - | > 120 | - | 40 |
| | 250 | 120 | > 120 | - | 120 |
| | 100 | 120 | > 120 | - | > 120 |
| | 50 | > 120 | - | - | - |
| B 1 | 500 | - | - | 5 | 120 |
| | 250 | 5 | 10 | 10 | 120 |
| | 100 | 10 | 20 | 20 | > 120 |
| | 50 | 10 | 60 | - | - |
| B 2 | 500 | - | - | 10 | 5 |
| | 250 | 2,5 | 20 | 40 | 10 |
| | 100 | 10 | 60 | > 120 | 120 |
| | 50 | 20 | 120 | - | - |
| B 3 | 1000 | - | - | - | 5 |
| | 750 | - | - | - | 5 |
| | 500 | - | - | 5 | 5 |
| | 250 | 10 | 10 | 10 | - |
| | 100 | 20 | 20 | 120 | - |
| | 50 | 60 | > 120 | - | - |

/15

Tabelle III - Fortsetzung

| Substanz | ppm | Abtötungszeit in Minuten | | | |
|---|---|---|---|---|---|
| | | 1 | 2 | 3 | 4 |
| B 4 | 1000 | - | - | - | 2,5 |
| | 750 | - | - | - | 2,5 |
| | 500 | - | - | 5 | 5 |
| | 250 | 10 | 10 | 10 | - |
| | 100 | 40 | 40 | 60 | - |
| | 50 | > 120 | > 120 | - | - |
| B 5 | 500 | - | - | 20 | 40 |
| | 250 | 20 | > 120 | > 120 | > 120 |
| | 100 | 60 | > 120 | > 120 | > 120 |
| | 50 | 120 | > 120 | - | - |
| B 6 | 500 | - | > 120 | > 120 | 40 |
| | 250 | 20 | > 120 | > 120 | 60 |
| | 100 | 40 | > 120 | > 120 | > 120 |
| | 50 | 60 | - | - | - |
| C 1 | 1000 | - | - | 2,5 | - |
| | 750 | - | - | 5 | 2,5 |
| | 500 | 5 | 10 | > 120 | 2,5 |
| | 250 | 10 | 20 | - | 10 |
| | 100 | 60 | > 120 | - | - |
| C 2 | 1000 | - | - | 10 | - |
| | 750 | - | - | 20 | 2,5 |
| | 500 | 2,5 | 20 | > 120 | 2,5 |
| | 250 | 5 | 20 | - | 20 |
| | 100 | 120 | > 120 | - | - |

Tabelle III - Fortsetzung

| Substanz | ppm | Abtötungszeit in Minuten 1 | 2 | 3 | 4 |
|---|---|---|---|---|---|
| C 3 | 1000 | - | - | - | - |
| | 750 | - | - | - | 2,5 |
| | 500 | 5 | 5 | - | 2,5 |
| | 250 | 40 | 20 | - | 20 |
| | 100 | > 120 | > 120 | - | - |
| C 4 | 1000 | - | - | 60 | - |
| | 750 | - | - | - | 10 |
| | 500 | 120 | 120 | - | 10 |
| | 250 | 120 | > 120 | - | 60 |
| | 100 | > 120 | > 120 | - | - |
| C 5 | 1000 | - | - | 10 | - |
| | 750 | - | - | - | 10 |
| | 500 | 2,5 | 60 | - | 10 |
| | 250 | 120 | > 120 | - | 40 |
| | 100 | 120 | > 120 | - | - |
| C 6 | 1000 | - | - | 20 | - |
| | 750 | - | - | - | 10 |
| | 500 | - | > 120 | - | 10 |
| | 250 | 10 | > 120 | - | 40 |
| | 100 | 60 | > 120 | - | - |
| | 50 | > 120 | - | - | - |
| D 1 | 500 | - | - | 5 | 60 |
| | 250 | 5 | 10 | 10 | 120 |
| | 100 | 10 | 10 | 60 | > 120 |
| | 50 | 20 | 120 | - | - |

/17

Tabelle III - Fortsetzung

| Substanz | ppm | Abtötungszeit in Minuten 1 | 2 | 3 | 4 |
|---|---|---|---|---|---|
| D 2 | 500 | - | - | 10 | 10 |
| | 250 | 2,5 | 5 | 20 | 20 |
| | 100 | 2,5 | 5 | 20 | 40 |
| | 50 | 10 | 10 | - | - |
| D 3 | 500 | - | - | 2,5 | 5 |
| | 250 | 5 | 5 | 5 | 10 |
| | 100 | 10 | 10 | 40 | 40 |
| | 50 | 20 | 20 | - | - |
| D 4 | 500 | - | - | 10 | 40 |
| | 250 | 20 | 20 | 20 | 60 |
| | 100 | 40 | 40 | 60 | > 120 |
| | 50 | 120 | 120 | - | - |
| D 5 | 500 | - | - | 2,5 | 40 |
| | 250 | 10 | 10 | 20 | 60 |
| | 100 | 40 | 60 | > 120 | > 120 |
| | 50 | 120 | 120 | - | - |
| D 6 | 1000 | - | - | 10 | - |
| | 750 | - | - | 10 | - |
| | 500 | - | 10 | - | 60 |
| | 250 | 10 | 20 | - | 60 |
| | 100 | 40 | 120 | - | > 120 |
| | 50 | > 120 | - | - | - |
| E 1 | 500 | - | - | 20 | 10 |
| | 250 | 2,5 | 10 | 40 | 10 |
| | 100 | 2,5 | 10 | 40 | 60 |
| | 50 | 10 | 20 | - | - |

Tabelle III - Fortsetzung

| Substanz | ppm | Abtötungszeit in Minuten | | | |
|---|---|---|---|---|---|
| | | 1 | 2 | 3 | 4 |
| E 2 | 500 | - | - | 5 | 5 |
| | 250 | 2,5 | 10 | 10 | 4 |
| | 100 | 5 | 10 | 10 | 120 |
| | 50 | 20 | 20 | - | - |
| E 3 | 500 | - | - | - | 10 |
| | 250 | 2,5 | 10 | 10 | 20 |
| | 100 | 10 | 10 | 20 | 20 |
| | 50 | 20 | 40 | 40 | - |
| E 4 | 500 | - | - | 20 | > 120 |
| | 250 | 20 | 60 | 60 | > 120 |
| | 100 | 120 | 120 | 120 | > 120 |
| | 50 | 120 | > 120 | > 120 | - |
| E 5 | 500 | - | - | 5 | 60 |
| | 250 | 20 | 40 | 40 | 120 |
| | 100 | 120 | 120 | 120 | > 120 |
| | 50 | 120 | > 120 | - | - |
| E 6 | 500 | - | - | - | 20 |
| | 250 | 10 | 40 | 10 | 60 |
| | 100 | 20 | 120 | 20 | > 120 |
| | 50 | 40 | 120 | 40 | - |
| F 1 | 1000 | - | - | 10 | - |
| | 750 | - | - | 10 | - |
| | 500 | - | - | 20 | 60*) |
| | 250 | 5 | 20 | - | 120 |
| | 100 | 20 | 20 | - | > 120 |
| | 50 | 40 | 40 | - | - |

/19

Tabelle III - Fortsetzung

| Substanz | ppm | Abtötungszeit in Minuten | | | |
|---|---|---|---|---|---|
| | | 1 | 2 | 3 | 4 |
| F 2 | 1000 | - | - | 10 | - |
| | 750 | - | - | 10 | - |
| | 500 | - | - | 20 | 40 |
| | 250 | 40 | 40 | - | 60 |
| | 100 | 40 | 60 | - | > 120 |
| | 50 | 60 | > 120 | - | - |
| F 3 | 1000 | - | - | 20 | - |
| | 750 | - | - | 60 | - |
| | 500 | - | - | 60 | 40 |
| | 250 | 60 | 120 | - | 40 |
| | 100 | 120 | > 120 | - | > 120 |
| | 50 | > 120 | > 120 | - | - |
| F 4 | 1000 | - | - | 120 | - |
| | 750 | - | - | > 120 | - |
| | 500 | - | - | > 120 | > 120 |
| | 250 | 120 | > 120 | - | > 120 |
| | 100 | 120 | > 120 | - | > 120 |
| | 50 | 120 | > 120 | - | - |
| F 5 | 1000 | - | - | 120 | - |
| | 750 | - | - | 120 | - |
| | 500 | - | - | > 120 | 20 |
| | 250 | 120 | 120 | - | 60 |
| | 100 | 120 | 120 | - | > 120 |
| | 50 | 120 | > 120 | - | - |

/20

Tabelle III - Fortsetzung

| Substanz | ppm | Abtötungszeit in Minuten 1 | 2 | 3 | 4 |
|---|---|---|---|---|---|
| F 6 | 1000 | - | - | 60 | - |
| | 500 | - | - | - | 120 |
| | 250 | 40 | 40 | - | 120 |
| | 100 | 60 | 60 | - | 120 |
| | 50 | 120 | >120 | - | - |
| G 1 | 1000 | - | - | 2,5 | - |
| | 750 | - | - | 2,5 | - |
| | 500 | - | - | 2,5 | 5 |
| | 250 | 5 | 5 | - | 5 |
| | 100 | 10 | 10 | - | 20 |
| | 50 | 20 | 20 | - | - |
| G 2 | 1000 | - | - | 2,5 | - |
| | 750 | - | - | 2,5 | - |
| | 500 | - | - | 2,5 | 5 |
| | 250 | 5 | 5 | - | 5 |
| | 100 | 10 | 10 | - | 20 |
| | 50 | 20 | 20 | - | - |
| G 3 | 1000 | - | - | 2,5 | - |
| | 750 | - | - | 2,5 | - |
| | 500 | - | - | 2,5 | 2,5 |
| | 250 | 5 | 5 | - | 2,5 |
| | 100 | 120 | 120 | - | 5 |
| | 50 | > 120 | > 120 | - | - |

Tabelle III - Fortsetzung

| Substanz | ppm | Abtötungszeit in Minuten | | | |
|---|---|---|---|---|---|
| | | 1 | 2 | 3 | 4 |
| G 4 | 1000 | - | - | 2,5 | - |
| | 750 | - | - | 5 | - |
| | 500 | - | - | 5 | 5 |
| | 250 | 10 | 60 | - | 5 |
| | 100 | 20 | 60 | - | 10 |
| | 50 | 60 | 120 | - | - |
| G 5 | 1000 | - | - | 2,5 | - |
| | 750 | - | - | 2,5 | - |
| | 500 | - | - | 2,5 | 5 |
| | 250 | 2,5 | 20 | - | 10 |
| | 100 | 20 | 40 | - | 10 |
| | 50 | 40 | 60 | - | - |
| G 6 | 1000 | - | - | 2,5 | - |
| | 750 | - | - | 5 | - |
| | 500 | - | - | 10 | 60 |
| | 250 | 5 | 5 | - | > 120 |
| | 100 | 10 | > 120 | - | > 120 |
| | 50 | 60 | > 120 | - | - |

Beispiel 3

Die mikrobizide Wirkung der Substanzen A 1 und B 1 bei der Flächendesinfektion wurde nach dem Scheuerdesinfektionstest ermittelt. Dieses Prüfverfahren ist ebenfalls den Richtlinien für die Prüfung chemischer Desinfektionsmittel, herausgegeben von der Deutschen Gesellschaft für Hygiene und Mikrobiologie (1959) entnommen.

Als Modelle für kontaminierte Flächen dienten 6 x 6 cm große Stücke von lackierten Holzplatten und PVC-Fußbodenplatten. Zur Kontamination der Flächen wurden Suspensionen von

1) Staphylycoccus aureus $> 10^8$ Keime/ml
2) Candida albicans $> 10^8$ Keime/ml

verwendet. Auf jede Testfläche wurde 0,1 ml Keimsuspension pipettiert und mit Hilfe eines Glasspatels gleichmäßig verteilt. Nach dem Antrocknen der Keimsuspension wurden die Testflächen mit Hilfe eines Wattetupfers gleichmäßig mit der Desinfektionslösung benetzt.

Zur Herstellung der Desinfektionslösung dienten Konzentrate folgender Zusammensetzung:

10 Gew.-% Wirkstoff (Substanz A 1 oder B 1)
10 Gew.-% $C_{12}$-$C_{14}$-Fettalkohol + 7 Mol Äthylenoxid
10 Gew.-% Äthanol
70 Gew.-% Wasser

Diese Konzentrate wurden zum Einsatz gegen Staphylococcus aureus im Verhältnis 1 : 100 zum Einsatz gegen Candida albicans im Verhältnis 1 : 50 mit Wasser verdünnt.

Nach Einwirkungszeiten von 1, 2, 4 und 6 Stunden wurde jeweils ein Viertel der behandelten Flächen mit sterilen Tupfern abgerieben, die durch kurzes Eintauchen in Bouillon angefeuchtet waren. Die Tupfer wurden auf Bouillon-Agarplatten (Merck-Standard-I-Bouillon + 3 Gew.-% Tween 80 + 0,3 Gew.-% Lecithin) ausgestrichen. Die Agarplatten wurden bei Staphylococcus aureus 48 Stunden bei 37° C oder 310 K, bei Candida albicans 72 Stunden bei 30° C oder 303 K bebrütet.

Die Auswertung ergab die in Tabelle IV wiedergegebenen Abtötungszeiten.

Tabelle IV

Abtötungszeit der Produkte A 1 und B 1 in h

| Produkt | ppm | Abtötungszeit in h<br>Testkeim<br>1 | 2 |
|---|---|---|---|
| A 1 | 1000 | 6 | - |
| | 2000 | - | 6 |
| B 1 | 1000 | 6 | - |
| | 2000 | - | 4 |

Beispiel 4

Nachfolgend werden einige Beispiele für Zubereitungen angegeben, in die die erfindungsgemäß zu verwendenden

Substanzen eingearbeitet wurden:

Antiseptisches Reinigungsmittel für Wäschereien

| | | |
|---|---|---|
| Fettalkohol + 5 ÄO | 7 | Gew.-Teile |
| Fettalkohol + 10 ÄO | 7 | " " |
| Natriumtripolyphosphat | 32 | " " |
| Natriumcarbonat | 9 | " " |
| Natriumsulfat | 13 | " " |
| Wasserglas | 5 | " " |
| Natriumcarboxymethylcellulose | 1 | " " |
| Produkt D 4 | 7 | " " |
| Wasser | 19 | " " |

Desinfizierende Handwaschpaste

| | | |
|---|---|---|
| Dodecyldimethylaminoxid | 10 | Gew.-Teile |
| Kokosfettsäuremonoäthanolamid | 3 | " " |
| Bimsstein fein gemahlen | 50 | " " |
| Tripolyphosphat | 5 | " " |
| Produkt C 2 | 0,5 | " " |
| Glycerin | 3,5 | " " |
| Carboxymethylcellulose, Na-salz | 0,5 | |
| Wasser | 23 | " " |

Desinfizierender Reiniger für harte Oberflächen

| | | |
|---|---|---|
| Produkt B 1 | 13 | Gew.-Teile |
| Nonylphenol + 10 Mol Äthylenoxid | 13 | " " |
| Na-1-Hydroxyäthan-1,1-diphosphonat | 4 | " " |
| Isopropanol | 20 | " " |
| Wasser | 50 | " " |

"3-(omega-Aminoalkyl)-substituierte 1,3-Oxazolidine, deren Herstellung und Verwendung als antimikrobielle Mittel"

## Patentansprüche

1. Substituierte Oxazolidine der Formel

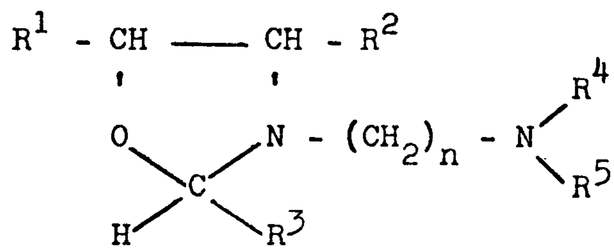

(I)

in der $R^1$ und $R^2$ Alkylreste mit 1 bis 17 Kohlenstoffatomen bedeuten, wobei die Summe der Kohlenstoffatome in $R^1$ und $R^2$ 8 bis 18 beträgt, $R^3$ Wasserstoff, einen Alkylrest mit 1 bis 5 Kohlenstoffatomen, einen gegebenenfalls durch Halogen, eine Hydroxyl- oder Methoxygruppe substituierten Phenylrest oder die Gruppe $C_6H_5CH{=}CH-$ darstellt, n eine ganze Zahl von 2 bis 6 und $R^4$ und $R^5$ unabhängig voneinander Wasserstoff oder einen Alkylrest mit 1 bis 3 Kohlenstoffatomen bedeuten.

2. Verwendung der substituierten Oxazolidine gemäß Anspruch 1 als antimikrobielle Mittel.

3. Verwendung der substituierten Oxazolidine gemäß Anspruch 1 nach Anspruch 2 zur Herstellung von antimikrobiellen Mitteln in Mengen von 0,1 bis 25 Gew.-%, vorzugsweise 0,5 bis 20 Gew.-%, bezogen auf das gesamte antimikrobielle Mittel.

/2

4. Verwendung der substituierten Oxazolidine gemäß Anspruch 1 nach Anspruch 2 zur Konservierung in Mengen von 0,1 bis 2 Gew.-%, bezogen auf das gesamte zu konservierende Produkt.

0006169

Nummer der Anmeldung

Europäisches Patentamt

# EUROPÄISCHER RECHERCHENBERICHT

EP 79 101 727.0

| Kategorie | EINSCHLÄGIGE DOKUMENTE<br>Kennzeichnung des Dokuments mit Angabe, soweit erforderlich, der maßgeblichen Teile | betrifft Anspruch |
|---|---|---|
| | DE - A - 2 338 516 (HENKEL)<br>* Ansprüche *<br>-- | 1,2 |
| | DE - A - 2 530 243 (HENKEL)<br>* Anspruch 2 *<br>-- | 2 |
| | DE - A - 2 512 980 (COMMERCIAL SOLVENTS)<br>* Anspruch 1 *<br>-- | 2 |
| | FR - A - 2 332 985 (BAYER)<br>* Anspruch 5 *<br>-- | 2 |
| A | GB - A - 1 415 566 (HENKEL)<br>-- | |
| A | US - A - 4 092 144 (IMC CHEMICAL GROUP)<br>---- | |

KLASSIFIKATION DER ANMELDUNG (Int.Cl.3)

C 07 D 263/04
A 01 N 43/76
C 07 D 263/06

RECHERCHIERTE SACHGEBIETE (Int. Cl.3)

A 61 L 13/00
C 07 D 263/04
C 07 D 263/06

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 03-09-1979 | FROELICH |